# FASCICULE DE BREVET EUROPEEN

(11) **EP 2 174 144 B1**
(45) Date de publication et mention de la délivrance du brevet: **11.04.2018**
(21) Numéro de dépôt: 08827979.9
(22) Date de dépôt: 29.07.2008
(51) Int. Cl.: G01N 1/38, G01N 30/18, G01N 33/49, G01N 35/10

(54) **SYSTÈME DE DISTRIBUTION COMPORTANT UN DISPOSITIF DE PRÉPARATION ET DE DISTRIBUTION FRACTIONNÉE D'ÉCHANTILLONS D'UN FLUIDE ET PROCÉDÉ ASSOCIÉ**
AUSGABESYSTEM MIT EINER VORRICHTUNG ZUR HERSTELLUNG UND FRAKTIONIERTEN AUSGABE VON FLÜSSIGKEITSPROBEN UND ENTSPRECHENDES VERFAHREN
DISPENSING SYSTEM INCLUDING A DEVICE FOR THE PREPARATION AND FRACTIONATED DISPENSING OF FLUID SAMPLES AND RELATED METHOD

(30) Priorité: 03.08.2007 FR 0756922
(43) Date de publication de la demande: 14.04.2010
(73) Titulaire: HORIBA ABX SAS, 34000 Montpellier (FR)
(72) Inventeur: LE COMTE, Roger, F-34470 Perols (FR); COUDERC, Guilhem, F-34430 Saint Jean De Vedas (FR); MORENO, Paul, F-34070 Montpellier (FR)
(74) Mandataire: Boura, Olivier
(86) Numéro de dépôt international: PCT/FR2008/051428
(87) Numéro de publication internationale: WO 2009/024710

(56) Documents cités:
- EP-A2- 1 275 957
- WO-A-2005/010488
- WO-A-2008/012445
- DE-A1- 2 220 465
- FR-A- 2 767 583
- FR-A1- 2 266 886
- FR-A1- 2 382 004
- GB-A- 2 025 900
- JP-U- S6 384 562
- JP-U- S61 193 372
- US-A- 3 719 086
- US-A- 4 888 998
- US-A- 5 853 665
- US-A1- 2006 099 716

## Description

La présente invention concerne le domaine de l'analyse de fluides, qu'ils soient biologiques ou non.

Le domaine de l'invention est en particulier celui de l'analyse de fluides biologiques tels que le sang, qu'ils soient d'origine humaine ou animale, et notamment le domaine des analyses hématologiques et la conception de systèmes et de procédés pour réaliser de telles analyses de façon automatique.

Plus particulièrement, la présente invention concerne un dispositif de préparation et de distribution fractionnée d'échantillons sanguins pour des automates d'analyses, ainsi qu'un système automatique de prélèvement, de préparation et de distribution fractionnée d'échantillons sanguins comportant un tel dispositif, et un procédé de préparation et de distribution d'échantillons sanguins à l'aide d'un tel système.

Dans la présente demande, on entend par les termes "échantillon sanguin", un volume de sang provenant d'une prise de sang réalisée sur un patient, mélangé à un anti-coagulant et contenue dans un récipient, tel qu'un tube ouvert ou fermé par un bouchon, et n'ayant subi ni traitement, ni altération. On parle alors, traditionnellement, d'échantillon de sang dit total ou complet.

Les analyses d'échantillons sanguins permettent de mesurer différents paramètres et de comptabiliser un certain nombre d'éléments constitutifs du sang permettant de renseigner l'état de santé des patients.

De tels paramètres sont, notamment, les globules rouges et blancs, l'hémoglobine ou encore les plaquettes.

Ces analyses sont réalisées à l'aide d'appareils automatiques (des analyseurs) incorporant des systèmes de prélèvement et de mesure automatiques permettant de déterminer la teneur des différents éléments recherchés dans le sang des patients.

Afin de mesurer les paramètres recherchés dans un échantillon sanguin, il est nécessaire de fractionner ledit échantillon en petites portions de volume déterminé, généralement appelées aliquotes. Ces aliquotes sont préparées (en pratique mélangées) avec différents réactifs pour permettre la détermination, notamment par des systèmes de mesure optiques, de la valeur des paramètres de l'échantillon de sang dans les aliquotes réalisées à partir de l'échantillon de sang de départ, et, ainsi, procurer des résultats d'analyse pour l'ensemble des éléments recherchés.

Afin d'optimiser les cadences d'analyses, mais, également, de réduire au maximum les volumes de réactifs utilisés, les différentes aliquotes de sang nécessaires pour réaliser les analyses sont obtenues à partir d'un unique échantillon de sang prélevé en une seule fois dans un tube par des moyens de prélèvement appropriés.

On connaît, à ce jour, différents types de dispositifs pour réaliser le fractionnement d'un échantillon sanguin en de multiples aliquotes et mélanger celles-ci avec des réactifs appropriés aux différentes analyses à réaliser.

On connaît, en premier lieu, des vannes d'échantillonnage, tels que, par exemple, enseignées par la demande de brevet français FR 2 622 692 au nom de la demanderesse.

De telles vannes d'échantillonnage permettent un fonctionnement des automates d'analyses à des cadences élevées et sont, traditionnellement, réservées aux appareils de haut de gamme car ces vannes sont chères à fabriquer et à régler.

De plus, les vannes d'échantillonnage présentent l'inconvénient de nécessiter une maintenance régulière et complexe qui renchérit leur coût d'exploitation. Par ailleurs, les vannes d'échantillonnage présentent également l'inconvénient de nécessiter des volumes de sang supérieurs aux volumes théoriquement nécessaires pour réaliser les analyses.

D'autres systèmes de fractionnement d'un échantillon de sang ont été développés. On peut citer par exemple les documents US 5,254,313 ou EP 0 543 544 déposés par la société TOA Medical. Dans ces deux cas, le prélèvement de l'échantillon de sang se fait dans un tube ouvert, il n'existe pas de système de perçage du bouchon. Les fractions (ou aliquotes) de sang sont mélangées au(x) réactif(s) dans une (ou plusieurs) cuve(s) de mélange. Enfin, c'est une partie mobile de la vanne qui permet l'aliquotage.

On peut encore citer le brevet WO 2005/010488 déposé par la société Dade Behring, dans lequel le prélèvement peut se faire dans un tube fermé par un bouchon. Mais là encore, le mélange échantillon/réactif se fait dans une cuve de mélange. De plus, l'aiguille de prélèvement doit se déplacer horizontalement au dessus de la cuve de mélange. US 3719086 A, JP S6384562 U, FR 2382004 A1, JP S61193372 U, FR 2266886 A1, DE 2220465 A1 et EP 1275957 A2 décrivent également des dispositifs de préparation et de distribution d'échantillons.

On connaît également un autre type de système de fractionnement d'un échantillon sanguin en de multiples aliquotes, décrit dans la demande de brevet EP 0913680 A1 au nom de la demanderesse.

Le dispositif décrit dans ce document comporte un organe de prélèvement de sang composé d'un premier tube percuteur pour percer un tube, dans lequel un échantillon de sang doit être prélevé, et aérer ce tube, et une aiguille montée coaxialement dans le percuteur pour venir prélever un échantillon de sang dans le tube, puis ensuite distribuer différentes aliquotes de l'échantillon prélevées dans le tube, dans un flux de réactifs à l'intérieur de différentes cuves d'analyse. L'organe de prélèvement du système est relié à une seringue pour aspirer un volume déterminé de sang dans le tube de sang à analyser, puis refouler les aliquotes dans les différentes cuves d'analyse en mélange avec les réactifs appropriés. Ledit organe de prélèvement est monté sur un dispositif mobile en translation verticalement et horizontalement par des moyens motorisés afin de pouvoir entrer dans les tubes de sang pour effectuer le prélèvement de l'échantillon de sang à analyser, puis ensuite distribuer une aliquote de sang dans chacune des cuves de mesure. Ce système permet ainsi de distribuer de multiples aliquotes d'un même échantillon de sang, de façon très précise, sans les inconvénients précédemment cités des vannes d'échantillonnage.

Cependant, le système de distribution fractionnée décrit dans EP 0913680 A1 n'est pas, non plus, entièrement satisfaisant.

En effet, les déplacements multiples de l'organe de prélèvement pour venir, d'une part, prélever l'échantillon de sang dans le tube puis, ensuite, distribuer les différentes aliquotes de cet échantillon dans les cuves d'analyse, nécessitent des réglages précis des systèmes mécaniques de transport de l'organe de prélèvement. On peut aussi noter une certaine variabilité dans le temps de la précision du positionnement de l'organe de prélèvement lors de la distribution des aliquotes de sang dans les cuves d'analyse par rapport aux orifices d'introduction des réactifs dans lesdites cuves.

Par ailleurs, dans certaines conditions très particulières, l'organe de prélèvement du système à perceur et l'aiguille de prélèvement coaxiaux peuvent subir des souillures du fait des opérations successives de perçage d'un même tube, avec des petites fractions de bouchons ou de sang séché qui peuvent s'intercaler entre l'aiguille et le corps interne du perceur et, ainsi, venir perturber la préparation des aliquotes.

Le but de la présente invention est de procurer un dispositif de préparation et de distribution fractionnée d'échantillons de fluide, et plus particulièrement d'échantillons de sang, qui permet de résoudre et, à tout le moins, d'atténuer les problèmes des dispositifs de distribution fractionnée connus de l'art antérieur.

Un autre but de l'invention est de procurer un dispositif de préparation et de distribution fractionnée qui soit plus simple et moins coûteux à réaliser que les systèmes connus à ce jour, et qui soit également plus simple à entretenir au cours du temps.

Un autre but de l'invention est de procurer un dispositif de distribution fractionnée d'échantillons sanguins qui permet de travailler à des cadences supérieures à celles autorisées par le système de distribution décrit dans EP 0913680 A1.

Un autre but de l'invention est également de procurer un dispositif de préparation et de distribution fractionnée d'échantillons sanguins qui permet de supprimer les problèmes de souillure des organes de prélèvement et de contamination des échantillons sanguins avant analyse, et qui permet également de limiter les volumes de réactifs utilisés pour les dilutions et analyses des échantillons sanguins.

Pour atteindre ces différents objectifs, la présente invention propose un système de prélèvement, de préparation et de distribution fractionnée d'échantillons d'un fluide tel que défini par la revendication 1.

Un procédé de préparation et de distribution fractionnée est également défini dans la revendication 10. Le corps du dispositif de préparation et de distribution est avantageusement formé d'un élément monobloc ou de plusieurs blocs d'un matériau biologiquement inerte tel que, par exemple, du Polyméthacrylate de méthyle (PMMA), du polypropylène, du polyéthylène, du verre, du Téflon ®, du PEEK ®, ou encore de l'inox, dans lequel sont usinés, injectés ou thermoformés ledit moyen de guidage et la ou les dites chambres de dilution.

Le dispositif de préparation et de distribution du système selon l'invention permet notamment une préparation et une distribution fractionnée d'échantillons de fluides tels que du sang par exemple sous formes de multiples aliquotes en totale sécurité opératoire, sans risque de souillure ni de blocage, avec un encombrement réduit et en assurant une grande précision de préparation.

La préparation des aliquotes de fluide se fait directement dans au moins une chambre de préparation intégrée au dispositif, et non plus dans des cuves réactionnelles annexes, avant d'être distribuée dans des moyens de récupération et/ou d'analyse. On supprime ainsi les problèmes de stabilisation liés aux divers déplacements des organes de prélèvement pour distribuer les aliquotes dans des cuves annexes. De plus l'invention permet aussi de réduire l'encombrement global de l'analyseur et le nombre de moteurs nécessaires au fonctionnement du dispositif. On réduit ainsi les risques liés aux éléments mécaniques.

Le dispositif de préparation et de distribution du système selon l'invention est également modulable en ce qu'il permet d'avoir, si nécessaire, plusieurs chambres de préparation empilées, de préférence les unes au-dessus des autres, et permettant ainsi de réaliser de multiples préparations et distributions quasi simultanées de différentes aliquotes d'un échantillon de fluide par simple déplacement de quelques millimètres d'un organe de prélèvement dans le conduit de guidage du dispositif.

Le dispositif de préparation et de distribution permet également de faire plusieurs préparations et distributions d'aliquotes dans la même chambre de préparation de façon successive.

Pour que l'organe de prélèvement traverse le dispositif de part en part avant de s'introduire dans le tube, il est nécessaire que le tube, récipient dans lequel se trouve le fluide à analyser, soit présenté au niveau de la surface du corps opposée à celle au niveau de laquelle l'organe de prélèvement pénètre dans le corps du dispositif. Typiquement, le tube sera présenté sous le dispositif en face de l'orifice du moyen de guidage sur la surface inférieure du dispositif alors que l'organe de prélèvement viendra s'introduire dans le moyen de guidage du dispositif au niveau de la surface supérieure du dispositif, traverser le dispositif avant de pénétrer dans le tube pour y aspirer le fluide à analyser.

Ainsi ce dispositif de préparation et de distribution ne comporte aucune pièce mobile, ce qui donne une plus grande robustesse au système dans son ensemble. Le système de l'invention permet, en effet, de guider un organe de prélèvement, traditionnellement une aiguille, selon un seul axe. L'organe de prélèvement ne se déplace ainsi plus de manière horizontale comme dans les dispositifs de distribution fractionnée de l'état de la technique pour prélever le fluide et distribuer les différentes aliquotes de fluide, et donc sans les inconvénients de ces systèmes.

Conformément à une autre caractéristique préférée de l'invention, la ou les chambre(s) de préparation est (sont) constituée(s) par un canal traversant le dispositif formant ainsi deux orifices, respectivement, l'orifice d'introduction et l'orifice de distribution de la chambre de préparation.

Les mélanges avec les réactifs se font ainsi directement dans le dispositif, dans la ou les chambre(s) de préparation et non plus dans des cuves annexes comme proposé dans l'art antérieur. Cette configuration permet de réduire l'encombrement global du dispositif, et de supprimer les risques de souillures des aliquotes préparées.

Toujours selon l'invention, la ou les chambre(s) de préparation est (sont) constituée(s) d'un dit canal orienté selon un axe de préférence perpendiculaire audit axe YY' dudit moyen de guidage.

Cette configuration facilite la distribution rapide des aliquotes de fluide dans la ou les chambre(s) de préparation et leur préparation avec les réactifs dans ladite chambre.

Conformément à une autre caractéristique avantageuse du dispositif de préparation et de distribution, celui-ci comporte des moyens de nettoyage d'un dit organe de prélèvement introduit dans le moyen de guidage.

Ces moyens de nettoyage comportent notamment une chambre de rinçage formée dans le prolongement du moyen de guidage, la chambre de rinçage communicant avec au moins un moyen d'introduction d'un liquide de rinçage et avec au moins un moyen de vidange de ladite chambre de rinçage et d'évacuation du liquide de rinçage.

Ces moyens de nettoyages permettent notamment d'éliminer sur le dit organe de prélèvement le maximum de souillures potentielles que ce soit du sang séché sur le bouchon lorsque le fluide à analyser est du sang, ou des particules de bouchon, lors des déplacements verticaux dudit organe de prélèvement dans le conduit de guidage du dispositif de l'invention.

Par ailleurs, les moyens de nettoyage comportent également, dans un mode de réalisation particulier du dispositif de l'invention, au moins un joint racleur disposé dans le prolongement du moyen de guidage, de manière à racler la surface externe du dit organe de prélèvement introduit dans ledit moyen de guidage du dispositif lors des mouvements en translation dudit organe de prélèvement dans ledit moyen de guidage.

Des systèmes assurant l'étanchéité entre les dites chambres de préparation et l'organe de prélèvement, sont inclus dans le dispositif afin de séparer physiquement chacune des chambres de préparation. Dans une réalisation particulière de l'invention ces systèmes d'étanchéité peuvent être des joints toriques.

Le système de prélèvement, préparation et distribution fractionnée d'échantillons de fluides de l'invention est ainsi un système de type "tout en un".

Ce système est ainsi éventuellement capable de percer le bouchon d'un tube de prélèvement, éventuellement de le ventiler pour le mettre à la pression atmosphérique, de prélever des aliquotes à partir d'un échantillon de fluide et de les préparer avec un(ou des) réactif(s) approprié(s), de distribuer les aliquotes ainsi préparées dans des cuves de récupération et/ou d'analyse, et de nettoyer l'ensemble du dispositif.

Les mélanges avec les réactifs se font directement dans le dispositif de préparation et de distribution, dans la ou les chambres de préparation.

De plus, les moyens de nettoyage du dispositif de préparation permettent d'éliminer le maximum de souillures potentielles que ce soit du sang séché sur le bouchon, ou des particules de bouchon à l'extrémité de l'organe de prélèvement.

Tous ces avantages permettent une réduction des coûts, une versatilité d'utilisation puisqu'on peut empiler plusieurs chambres de préparation, et enfin une souplesse d'intégration puisqu'il n'est pas indispensable que l'aiguille soit près des cuves pour distribuer les aliquotes de sang.

De plus, le volume des aliquotes peut être modifié en fonction des besoins des analyses.

Dernier avantage, même si la pointe de l'organe de prélèvement est souillée par des déchets, celle-ci n'est jamais en contact avec la chambre de préparation car l'orifice de distribution des aliquotes de fluide est situé latéralement sur l'organe de prélèvement, ce qui évite toute contamination.

Dans différentes configurations privilégiées, le système de l'invention peut comporter en outre les caractéristiques avantageuses suivantes :
- des moyens de distribution d'au moins un fluide de rinçage de l'organe de prélèvement, relié audit moyen d'introduction d'un fluide de rinçage dans la chambre de rinçage du dispositif de préparation et de distribution, et des moyens d'aspiration et de récupération du fluide de rinçage, reliés au moyen de vidange de la chambre de rinçage et d'évacuation du fluide de rinçage du dispositif de préparation et de distribution.

Le système de prélèvement, préparation et distribution fractionnée d'échantillons de fluides selon l'invention présenté ci-dessus facilite les opérations de distribution fractionnée d'échantillons de fluides, notamment de sang, à analyser dans les machines d'analyses automatiques.

Ce système permet notamment de procéder à une distribution fractionnée des échantillons sanguins selon les étapes suivantes :
a- on aspire un échantillon d'un fluide contenu dans un tube, dans l'organe de prélèvement du système, l'organe de prélèvement (10) ayant été guidé préalablement en translation selon l'axe YY', typiquement vers le bas, au travers du dispositif (1), jusqu'à ce que l'extrémité de l'organe de prélèvement (10) s'introduise dans le tube (T) contenant le fluide, puis
b- on déplace, typiquement vers le haut, ledit organe de prélèvement en translation selon l'axe YY' du moyen de guidage du dispositif de préparation et de distribution, jusqu'à amener ledit orifice de l'organe de prélèvement dans au moins une chambre de préparation du dispositif de préparation et de distribution, puis enfin
c- on distribue, simultanément ou non, une aliquote de l'échantillon de fluide prélevé par l'orifice de l'organe de prélèvement et un volume déterminé d'un réactif dans ladite chambre de préparation du dispositif de préparation et de distribution, ladite aliquote de fluide et ledit volume déterminé de réactif étant mélangés puis évacués à co-courant sous la pression du fluide réactif vers un moyen de récupération et/ou d'analyse dudit mélange par l'orifice de distribution du dispositif de préparation et de distribution.

Le procédé de préparation et de distribution fractionnée de l'invention prévoit également qu'après prélèvement de l'échantillon de fluide dans un tube et avant distribution d'une dite aliquote dans ladite chambre de préparation du dispositif de préparation et de distribution, on nettoie l'organe de prélèvement ayant été en contact avec le fluide par l'intermédiaire des moyens de nettoyage du dispositif de préparation et de distribution. De la sorte on supprime, ou du moins on limite à un minimum les risques de souillures de l'organe de prélèvement et donc de perturbations des analyses.

Enfin, lorsque les tubes dans lesquels les échantillons de fluide doivent être prélevés sont dépressurisés, le procédé de l'invention prévoit également qu'avant prélèvement d'un dit échantillon de fluide dans un dit tube, fermé par un bouchon, on équilibre, le cas échéant, la pression à l'intérieur dudit tube, par l'intermédiaire dudit organe de prélèvement.

D'autres caractéristiques et avantages de la présente invention ressortiront mieux à la lecture de la description qui va suivre, faite de manière illustrative et non limitative, en référence aux dessins annexés sur lesquels :
- la figure 1A représente, en perspective, un dispositif de préparation et de distribution fractionnée dans un mode de réalisation préféré, conformément à l'invention ;
- la figure 1B représente, vu en coupe selon un plan longitudinal médian, le dispositif de préparation et de distribution représenté à la figure 1A ;
- la figure 2 représente un système de prélèvement, préparation et distribution fractionnée d'échantillons de sang, conforme à la présente invention, le système incorporant un dispositif de préparation et de distribution fractionnée tel que représenté aux figures 1A et 1B ;
- les figures 3A à 3C représentent le mode de fonctionnement du système de prélèvement, préparation et distribution fractionnée d'échantillons sanguins représenté à la figure 2 selon le procédé de la présente invention.

Le dispositif, le système comportant ledit dispositif ainsi que le procédé utilisant ledit système sont plus particulièrement destinés à l'analyse hématologique, mais peuvent tout aussi bien servir à toute sorte d'analyses de fluides. Toutefois, la description qui va suivre ne se focalisera que sur l'application dans le domaine de l'analyse hématologique d'échantillons de sang.

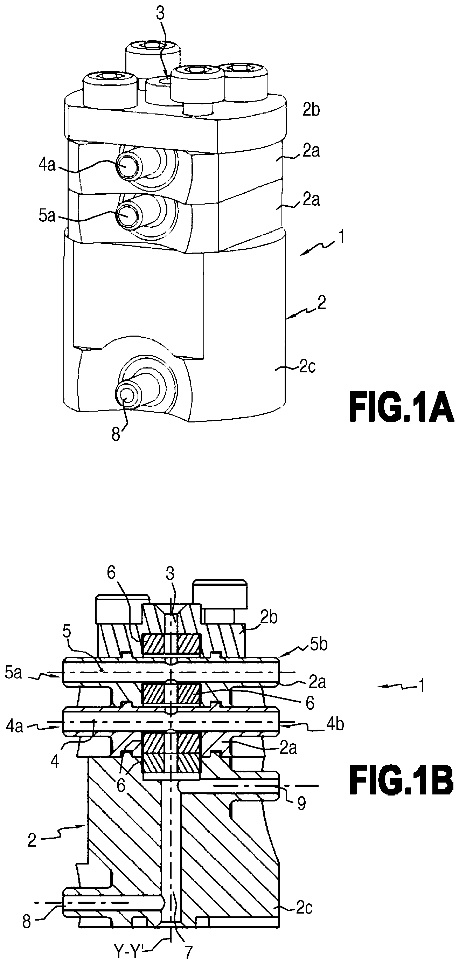

En référence tout d'abord aux figures 1A et 1B, on a représenté, conformément à un premier objet de l'invention, un dispositif 1 de préparation et de distribution fractionnée d'échantillons permettant de réaliser, de manière automatique, la division d'échantillons en une pluralité d'aliquotes et leur préparation et distribution pour mesurer différents paramètres.

Ce dispositif 1 comporte essentiellement un corps 2, percé selon un axe YY' d'un conduit cylindrique 3, débouchant au niveau des surfaces supérieure et inférieure du corps 2 du dispositif.

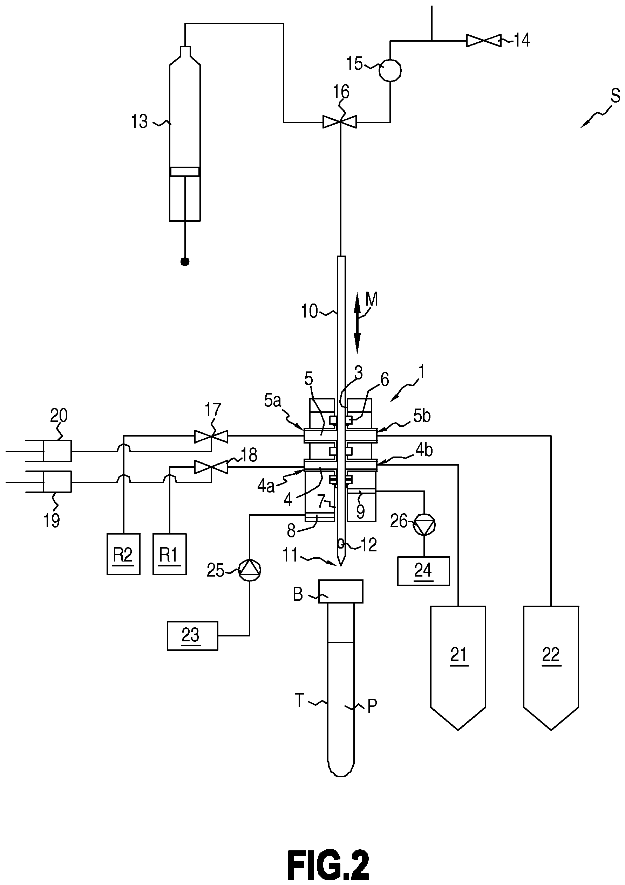

Ce conduit 3 débouchant constitue un moyen de guidage adapté pour recevoir et guider en translation, selon l'axe YY' du dispositif 1, un organe de prélèvement d'échantillon tel qu'une aiguille 10 comme représenté et décrit par la suite en référence aux figures 2 et suivantes.

En sus du conduit 3, le corps 2 du dispositif 1 comporte également des chambres de préparation 4, 5, superposées l'une sur l'autre et formées chacune par un conduit traversant le corps 2, selon deux axes, de préférence parallèles entre eux, et sécants de l'axe YY' du dispositif 1.

Les chambres de préparation 4, 5 sont toutes deux sécantes du conduit vertical 3 de guidage percé verticalement dans le corps 2 du dispositif 1 et communiquent donc, en l'absence d'organe de prélèvement, avec ce dernier.

De plus, les chambres de préparation 4 et 5 comportent chacune à leurs extrémités un premier orifice débouchant et un second orifice débouchant formant respectivement des orifices d'introduction 4a, 5a d'au moins un réactif d'analyse et des orifices de distribution 4b, 5b d'un mélange formé d'un dit réactif et d'une aliquote de sang préparée dans chaque chambre de préparation 4, 5.

Pour assurer une parfaite étanchéité entre chacune des chambres de préparation 4, 5 et le conduit de guidage 3 lorsqu'un organe de prélèvement tel qu'une aiguille est introduite dans ce conduit de guidage, le dispositif 1 comporte en son corps 2 des joints d'étanchéité 6 disposés de part et d'autre des lumières de raccordement entre le conduit 3 de guidage et chacune des chambres de préparation 4, 5. On remarque aussi qu'il est utile que les joints d'étanchéité, au moins celui ou ceux situé(s) sur le bas de la ou des chambre(s) de préparation, présentent une élasticité telle qu'en l'absence de l'organe de prélèvement, l'orifice central du joint se referme. Ainsi, on évite toute perte de fluide vers le bas quand l'aiguille de prélèvement n'est plus engagée dans le joint et toute contamination éventuelle. En l'absence de tels joints, il sera nécessaire que les séquences de manipulation du dispositif soit telles que l'aiguille bouche l'orifice du joint de la chambre de préparation concernée lors des manipulations de mélange dans cette chambre de préparation.

Formé dans le prolongement du conduit de guidage 3, toujours selon l'axe YY' du dispositif, le corps 2 du dispositif 1 comporte une chambre de rinçage 7 constituée d'un conduit débouchant sur le conduit de guidage 3 et communiquant avec celui-ci à son extrémité supérieure.

Cette chambre de rinçage 7 communique, à son extrémité inférieure, avec un conduit d'alimentation 8, percé dans la base du corps 2 du dispositif 1 et à son extrémité supérieure avec un conduit de vidange 9, également percé dans le corps 2 du dispositif.

Ces conduits 8, 9 permettent, respectivement, d'introduire dans la chambre de rinçage et d'en extraire un liquide de rinçage pour nettoyer l'extrémité d'un organe de prélèvement introduit dans le conduit de guidage 3 du dispositif lors de son déplacement en translation le long de l'axe YY' du dispositif 1 lors du fonctionnement dudit dispositif, comme il sera décrit par la suite.

De plus, le dispositif 1 comporte également un joint et, le cas échéant, un joint racleur au niveau de l'extrémité débouchante de la chambre 7 qui permet d'enlever toute souillure éventuellement déposée sur l'organe de prélèvement lors de son entrée dans la chambre 7. Ce joint racleur peut être de même nature que les joints d'étanchéité utilisés pour séparer physiquement les chambres de préparation, mais peut tout aussi bien être de nature différente.

Le corps 2 du dispositif 1 de préparation et de distribution d'échantillon fractionnée selon l'invention est avantageusement constitué d'un matériau inerte biologiquement. Ce matériau peut être du polypropylène, le Polyméthacrylate de méthyle (PMMA), du polyéthylène, du verre, de l'inox, du Téflon ®, du PEEK ® ou tout autre matériau bien connu de l'homme de l'art.

Par ailleurs, si le corps 2 du dispositif 1 de l'invention peut être réalisé d'un seul tenant en une pièce unique, par exemple par moulage, il est préférable, comme dans l'exemple de réalisation représenté sur les figures 1A et 1B, de réaliser le corps 2 sous forme modulable à partir de modules distincts, aptes à être assemblés les uns aux autres, tels que notamment des modules 2a pour réaliser une ou plusieurs chambres de préparation ainsi qu'un module 2b de couverture du ou des module(s) 2a procurant une chambre de préparation et d'un module 2c, dans lequel sont formés la chambre de rinçage 7, ses conduits d'alimentation et de vidange 8, 9, les modules 2b et 2c étant respectivement aptes à être emboîtés sous un module 2a par tout moyen d'emboîtement ou de liaison approprié, notamment tenon et mortaise ou rainure et languette formées ou niveau des surfaces d'emboîtement de chacun des modules 2a, 2b et 2c, et maintenus fermement ensemble par des vis d'assemblage.

Une construction modulaire du corps 2 du dispositif de préparation et de distribution fractionnée de la présente invention présente en particulier l'avantage de permettre une modularité fonctionnelle du dispositif 1 en fonction du type d'analyseur automatique dans lequel il doit être utilisé.

En particulier, une structure modulaire permet d'adapter le nombre de chambres de préparation du dispositif 1 en multipliant et empilant les uns sur les autres les modules 2a qui délimitent chacun une dite chambre.

De plus, la structure modulaire du corps présente également l'avantage de faciliter l'entretien du dispositif 1 en le rendant démontable, ce qui permet d'une part, de nettoyer régulièrement chacun des modules 2a, 2b, 2c mais, également, de changer facilement chacun des joints d'étanchéité 6 du dispositif qui, lorsque le corps a une structure modulaire, sont logés dans des lamages cylindriques coaxiaux aux conduits de guidage cylindriques 3 percés selon l'axe longitudinal YY' du dispositif.

Ainsi, lorsque un des joints d'étanchéité est défaillant, il suffit de désolidariser chacun des modules 2a, 2b et 2c du dispositif puis de retirer le joint défaillant 6 et le remplacer par un nouveau joint et remonter l'ensemble.

Si le corps 2 du dispositif 1 est réalisé d'un seul tenant, le système d'étanchéité pourra alors être procuré et réalisé par tout ou partie du monobloc 2, notamment en choisissant un matériau adapté pour la réalisation de celui-ci.

Le dispositif 1 de préparation et de distribution fractionnée d'échantillons sanguins de l'invention permet de réaliser de façon simple, sécurisée et rapide, le prélèvement d'un échantillon de sang dans un tube, ouvert ou fermé par un bouchon et de le diviser en une pluralité d'aliquotes de très faible volumes, de préparer ces différentes aliquotes à l'intérieur des chambres de préparation 4, 5 avec des volumes choisis de différents réactifs et de distribuer ces mélanges, vers des moyens de récupération et/ou d'analyse.

Le dispositif 1 des figures 1A et 1B a notamment été conçu par les demandeurs comme partie intégrante d'un système complet S de prélèvement, préparation et distribution fractionnée d'échantillons de fluides, notamment de sang, dont on veut réaliser l'analyse, tel que représenté à la figure 2.

Ce système S de prélèvement, préparation et distribution fractionnée comporte en premier lieu un dispositif 1 de préparation et distribution fractionnée, tel que décrit précédemment et représenté aux figures 1A et 1B dans le conduit de guidage 3 duquel est introduit un organe de prélèvement 10 prenant de préférence la forme d'une aiguille allongée, insérée selon l'axe YY' dans le conduit 3 du dispositif 1 et mobile en translation selon l'axe YY' dans ce conduit 3 par l'intermédiaire d'un dispositif de déplacement.

L'organe de prélèvement 10 comporte une première extrémité 11 pointue à proximité de laquelle est percé, légèrement en retrait par rapport à ladite extrémité pointue, un orifice de prélèvement et de distribution 12, de fait localisé latéralement sur l'organe de prélèvement.

L'aiguille de prélèvement 10, peut présenter un orifice latéral unique 12 mais peut également présenter plusieurs trous afin de constituer un premier filtre empêchant le passage de bouts de bouchons provenant du percement des bouchons B des tubes T.

La taille et la disposition de ces trous peuvent être variables et plusieurs configurations pourraient être envisagées. Toutefois, la surface du (ou des) trou(s) 12 ne doit pas être plus grande que la section des chambres de préparation.

L'organe de prélèvement peut aussi comporter des canaux multiples permettant pour certains d'entre eux de réaliser le prélèvement de l'échantillon et pour certains autres de réaliser l'aération du tube si nécessaire. De même, l'organe de prélèvement peut être constitué de plusieurs aiguilles de prélèvement.

A l'extrémité opposée de son extrémité pointue, l'organe de prélèvement 10 est relié par des moyens appropriés à un dispositif volumétrique d'aspiration et de refoulement de sang 13 tel que par exemple, une seringue motorisée.

Par ailleurs, le système S décrit à la figure 2, comporte également des moyens d'aération d'un tube de sang T. Ces moyens comportent une vanne de mise à l'air 14 connectée en série à un capteur de pression 15 lui-même relié à l'organe de prélèvement 10 *via* une vanne 16.

Ces moyens d'aération permettent, avantageusement et si nécessaire, de réaliser une mise à pression atmosphérique d'un tube T fermé par un bouchon B avant prélèvement d'un échantillon, puisqu'une dépression ou une surpression à l'intérieur du tube T peuvent perturber l'aspiration de l'échantillon par l'aiguille 10 dans le tube T.

Entre la seringue 13, les éléments 14, 15 d'aération et l'aiguille de prélèvement 10, est placée une vanne 16 formant un aiguillage hydraulique permettant d'établir alternativement selon un cycle déterminé, une communication entre l'aiguille de prélèvement 10 et la seringue 13 ou entre l'aiguille de prélèvement 10 et le système d'aération de tube formé des éléments 14, 15.

Ainsi que représenté sur cette figure, le tube T contenant un fluide P à analyser est placé sous le dispositif de préparation et distribution 1, ou du moins, du coté du dispositif 1 opposé au côté du dispositif 1 dans lequel vient pénétrer l'organe de prélèvement 10. Ici, le tube T est placé du coté de la surface inférieure du dispositif 1 ce qui est avantageux d'un point de vue pratique pour gérer l'amenée du tube en face de l'orifice inférieur du moyen de guidage du dispositif 1. Dans le cas contraire, qui reste possible mais moins aisé de mise en oeuvre, il faudrait que le tube soit renversé, l'organe de prélèvement remontant alors au travers du dispositif selon l'invention pour venir prélever l'échantillon puis redescendant pour le distribuer ensuite dans les chambres de préparation.

Les chambres de préparation 4, 5 du dispositif 1 sont reliées hydrauliquement au niveau de leur orifice respectif d'introduction de réactifs 4a, 5a à un réservoir R1, R2 de réactif. Entre lesdits réservoirs R1, R2 et les orifices 4a, 5a des chambres de préparation sont disposées des vannes 17, 18 permettant d'introduire un volume déterminé de réactif depuis chacun des réservoirs R1, R2, à l'aide par exemple de seringues motorisées 19, 20 dans les chambres de préparation 4, 5 pour mélanger ces réactifs avec une aliquote distribuée par l'aiguille de prélèvement dans lesdites chambres 4, 5 comme décrit par la suite.

Le mélange entre les aliquotes de sang et les réactifs se fait directement à la sortie de l'aiguille 10, dans le flux de réactif à l'intérieur des chambres de préparation 4, 5. Le dispositif 1 permet donc un meilleur mélange entre l'échantillon et les réactifs.

Au niveau de leur orifice de distribution 4b, 5b, les chambres de préparation 4, 5 du dispositif 1 sont reliées hydrauliquement à une cuve de récupération et/ou à des moyens d'analyse 21, 22 dans laquelle est distribué le mélange formé dans les chambres 4, 5 par un réactif et une aliquote de sang déposée au préalable par l'organe de prélèvement dans ladite chambre comme il sera explicité plus en détail par la suite.

Ainsi, sans avoir à déplacer le dispositif, les aliquotes mélangées aux réactifs sont distribuées dans les différentes cuves de récupération et/ou dans des moyens d'analyse 21, 22. L'aiguille de prélèvement 10 n'a pas à être déplacée au dessus de ces cuves, il faut juste un tuyau pour amener le sang mélangé au réactif jusque dans les cuves 21, 22 depuis les orifices de distribution 4b, 5b des chambres de dilutions 4, 5 du dispositif 1.

Le système S comporte également un réservoir de liquide de rinçage 23 relié au canal de rinçage 8 du dispositif 1 par l'intermédiaire de canalisations et d'une pompe de distribution 25 ainsi qu'un réservoir de récupération 24 relié au canal d'aspiration de liquide de rinçage 9 du dispositif 1 par l'intermédiaire d'une canalisation et d'une pompe d'aspiration 26.

Ces moyens de rinçage permettent ainsi d'introduire, depuis le réservoir 23, dans la chambre de rinçage 7 du dispositif 1 un liquide de rinçage afin de nettoyer l'organe de prélèvement 10 lors de sa remontée vers les chambres de préparation 4, 5 du dispositif 1 et d'aspirer simultanément le liquide de rinçage introduit, le cas échéant mélangé avec des morceaux de bouchon de tube par le canal 7 vers le réservoir de récupération 24.

De façon bien connue de l'homme de l'art, le système S et l'ensemble de ses éléments constitutifs, notamment les éléments mobiles de celui-ci peuvent être et sont en pratique pilotés et contrôlés par une unité de commande automatique non représentée sur les figures. Cette unité de commande peut notamment comporter un pupitre et des moyens de commande informatiques permettant de programmer le fonctionnement du système S et de chacun de ces composants, ainsi que généralement le fonctionnement de l'ensemble de l'analyseur automatique dans lequel le système S est installé et utilisé.

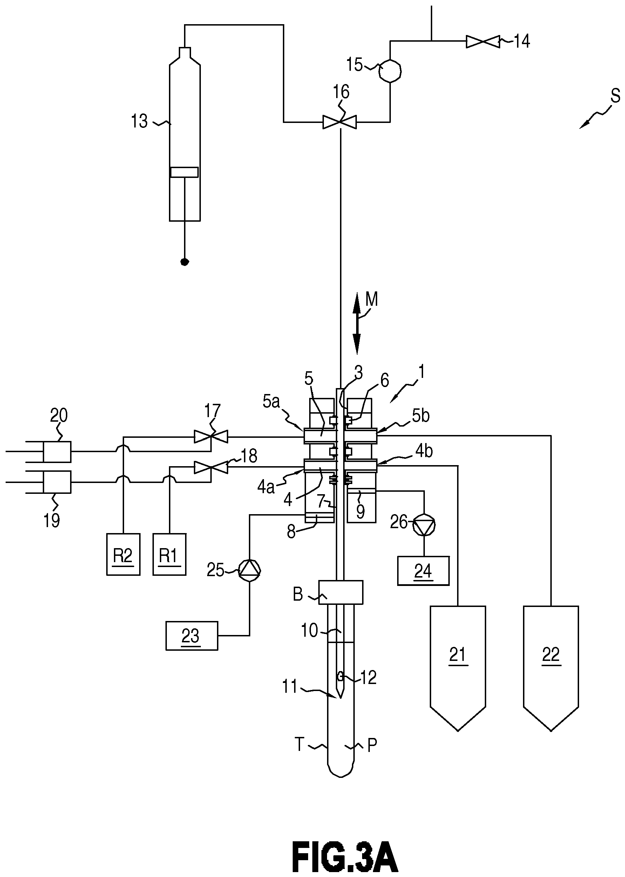

Le fonctionnement du dispositif 1 de préparation et de distribution fractionnée de la présente invention ainsi que celui du système S de prélèvement en préparation et distribution fractionnée incorporant ce dispositif va maintenant être décrit plus en détail, relativement aux figures 3A à 3C.

En référence tout d'abord à la figure 3A, on vient, dans un premier temps, prélever un échantillon de sang au moyen de l'aiguille de prélèvement 10, dans un tube T, fermé par un bouchon B contenant un prélèvement sanguin P. Il est à noter que le système S peut fonctionner également avec des tubes T non fermés par des bouchons B.

Pour ce faire, on déplace l'aiguille 10 en translation selon l'axe YY' du dispositif 1 dans le conduit de guidage 3 du dispositif 1 jusqu'à ce que l'extrémité pointue 11 traverse le bouchon B du tube T. La pression à l'intérieur du tube T est alors mesurée à l'aide du capteur de pression 15. La vanne 16 établit la communication entre l'orifice 12 de l'aiguille 10 et le capteur de pression 15. Le capteur de pression détermine, via l'orifice 12, la pression à l'intérieur du tube. Si le tube T est en dépression (cas où un tube n'a pas été débouché puis rebouché, ce qui est le plus courant), la vanne d'équilibrage 14 est ouverte pour faire un rééquilibrage de la pression à l'intérieur du tube T de sang.

Une fois la pression atmosphérique atteinte, la vanne 16 ferme la communication hydraulique entre l'aiguille 10 et les moyens d'aération 14, 15 et établit la liaison entre l'aiguille 10 et la seringue 13.

L'aiguille 10 est ensuite descendue puis immergée suffisamment dans le tube T pour permettre le prélèvement de l'échantillon par le ou les orifice(s) 12 de l'aiguille 10.

Puis, le piston de la seringue 13 est actionné pour prélever un volume V d'échantillon de sang à analyser.

Une fois ce prélèvement réalisé, l'aiguille 10 est remontée dans le dispositif 1. L'extrémité 11 de ladite aiguille pénètre alors dans la chambre de rinçage 7 du dispositif 1 pour y être nettoyée.

La pompe 25 est alors actionnée pour introduire dans la chambre de rinçage 7 par le canal 8 un flux de liquide de rinçage depuis le réservoir 23. En même temps, la pompe 26 est également actionnée pour générer une aspiration d'air et de liquide de rinçage dans le chambre 7 par le canal 9, ce qui crée une turbulence le long de l'aiguille de prélèvement 10 et ce qui permet un bon nettoyage de celle-ci, l'air et le liquide de rinçage aspirés par la pompe 26 étant recueillis dans le réservoir 24.

En cas de souillure résiduelle de l'aiguille, les joints 6 disposés avant la première chambre de préparation 4 permettent d'éliminer toute trace résiduelle de souillure sur l'aiguille de prélèvement. Il est à noter que les joints 6 peuvent être identiques ou différents des joints disposés entre chaque chambre de préparation.

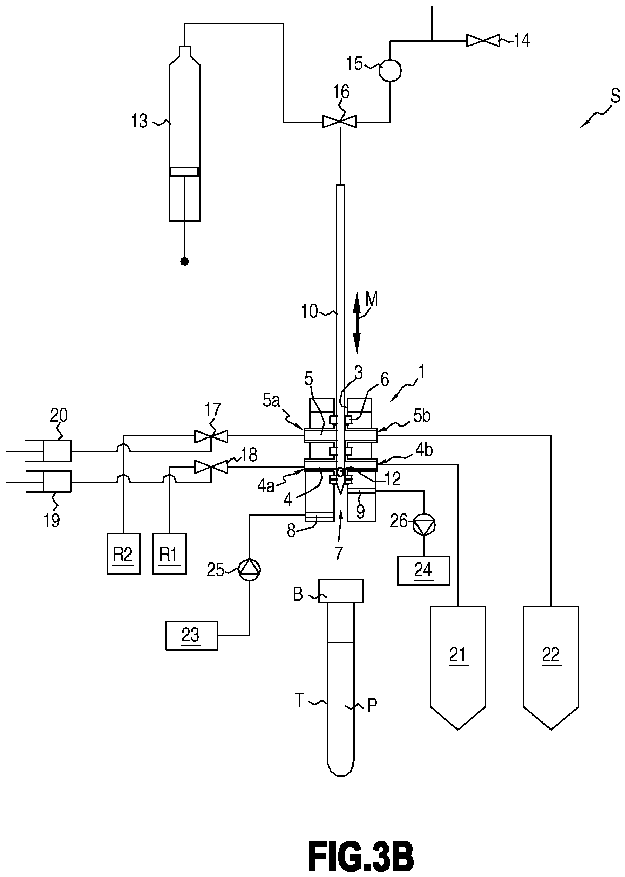

Une fois que l'aiguille de prélèvement 10 est nettoyée, celle-ci est alors une nouvelle fois remontée en translation dans le conduit 3 du dispositif 1, jusqu'à ce que l'orifice 12 de ladite aiguille soit positionné dans la première chambre de préparation 4 du dispositif 1, comme représenté à la figure 3B.

La seringue 13 pousse alors une première aliquote de sang a1 de volume déterminé dans la chambre de préparation 4.

Simultanément au dépôt de l'aliquote a1 dans la chambre 4, la vanne 18 reliée au réservoir R1 de réactif et à l'orifice d'introduction 4a de la chambre de préparation 4 est ouverte pour introduire un volume déterminé de réactif R1 aspiré puis poussé dans la chambre 4 par la seringue 19 et réaliser le mélange de l'aliquote a1 avec le réactif.

Ce mélange se réalise sous la pression d'introduction du réactif dans la chambre de préparation 4 depuis le réservoir R1 et, sous cette même pression, ce mélange est alors distribué par l'orifice de distribution 4b de la chambre de préparation 4 vers une cuve de récupération et/ou d'analyse 21.

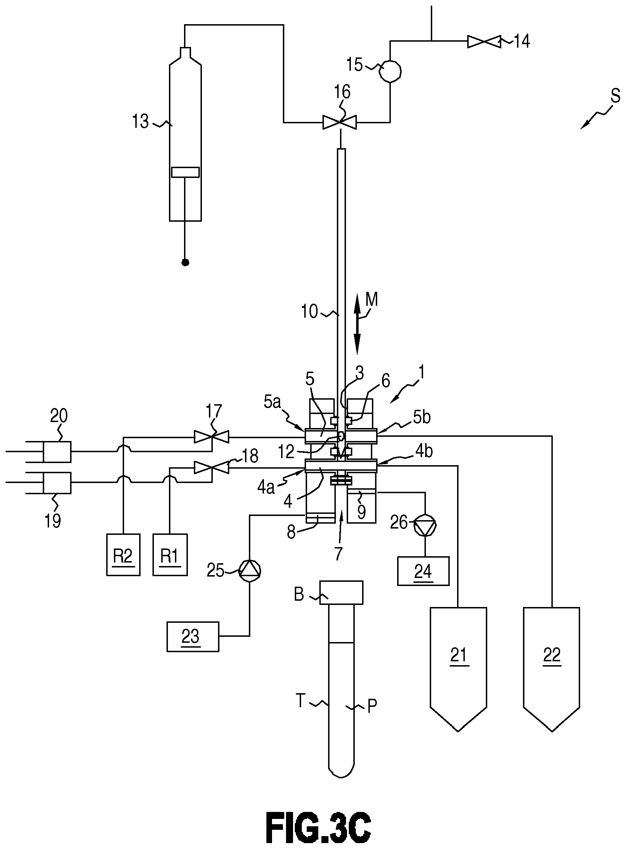

Dès que la distribution de la première aliquote a1 est terminée, l'aiguille de prélèvement 10 est remontée dans le conduit 3 du dispositif 1 jusqu'à ce que l'orifice 12 de ladite aiguille soit positionné dans la deuxième chambre de préparation 5, comme représenté à la figure 3C.

La distribution d'une seconde aliquote de sang a2 dans la chambre de préparation 5 est alors réalisée. Le piston de la seringue 13 pousse alors un volume déterminé de sang formant l'aliquote a2 dans la chambre 5 et en même temps est ouverte la vanne de distribution 19 pour introduire dans la chambre 5 par l'orifice 5a un volume déterminé de réactif depuis le réservoir R2, le réactif étant aspiré et poussé par une seringue motorisée 20.

Le mélange de l'aliquote de sang a2 avec le réactif est alors réalisé dans la chambre 5 tout comme sa distribution par l'orifice de distribution 5b vers une seconde cuve de récupération et/ou d'analyse 22.

L'aiguille de prélèvement 10 est ensuite redescendue dans le conduit 3 jusque dans la chambre de nettoyage 7 pour y être vidée, puis nettoyée et séchée, avant de recommencer un nouveau cycle de prélèvement/distribution d'un nouvel échantillon de sang.

Le dispositif 1 de préparation et distribution fractionnée de l'invention et le système S incorporant ce dispositif permettent ainsi de réaliser une distribution fractionnée en de multiples aliquotes d'un échantillon de fluide sans déplacement de l'aiguille entre le tube contenant le fluide à analyser et les cuves de réception et/ou d'analyse par simple alignement de l'orifice de l'aiguille 10 dans les chambres de préparation et de distribution du dispositif de préparation et de distribution fractionnée 1, objet de la présente invention.

On remarque enfin que diverses mises en oeuvre peuvent être réalisées selon les principes de l'invention. Notamment, l'axe YY' n'est pas nécessairement vertical, des réalisations fonctionnant selon le principe de l'invention peuvent aussi être mises en oeuvre dans lesquelles la translation de l'organe de prélèvement se ferait le long d'une autre direction que la direction verticale utilisée dans la description. L'invention concerne en effet tout système dans lequel un tube ou un récipient, le cas échéant adapté pour subir une perforation latérale, est aligné avec un moyen de guidage d'un dispositif de préparation et de distribution selon l'invention pour réaliser un prélèvement à l'aide d'un organe de prélèvement traversant ce dispositif, que l'axe de cet alignement soit vertical, horizontal ou fasse un angle quelconque. Les caractéristiques de l'invention, dans laquelle les chambres de préparation sont nécessairement étanches, autorisent ces réalisations.

## Revendications

1. Système (S) de prélèvement, de préparation et de distribution fractionnée d'échantillons d'un fluide, notamment de sang, comportant :
- un organe de prélèvement (10), notamment une aiguille, comportant une extrémité (11) apte à percer un bouchon (B) d'un tube (T) contenant un fluide, et au moins un orifice (12), apte à permettre l'aération dudit tube (T) et l'aspiration d'un échantillon de fluide dans le tube,
- au moins un moyen (13) d'aspiration et de refoulement de l'échantillon de fluide, relié à l'organe de prélèvement (10),
- au moins un dispositif (1) de préparation et de distribution comportant :
un moyen de guidage (3) constitué par un canal (3) qui est conformé pour recevoir et guider l'organe de prélèvement (10) en translation selon un axe YY' dans le canal (3) jusqu'à ce que l'extrémité de l'organe de prélèvement s'introduise dans le tube (T) contenant le fluide, et
au moins une chambre de préparation (4, 5), permettant la préparation d'une aliquote d'un échantillon de fluide distribuée dans la chambre par l'organe de prélèvement (10) dans un flux d'un réactif approprié, le moyen de guidage (3) traversant la chambre de préparation (4, 5) et communiquant avec elle, de manière à permettre la distribution d'une aliquote d'un échantillon de fluide dans la chambre par l'organe de prélèvement (10) dans une position déterminée de celui-ci dans le moyen de guidage, la chambre de préparation (4, 5) comportant un orifice d'introduction (4a, 5a) d'au moins un réactif (R1, R2) dans la chambre pour mélanger le réactif à une aliquote et au moins un orifice de distribution (4b, 5b) pour distribuer le mélange formé par l'aliquote et le réactif vers des moyens de récupération et/ou d'analyse (21,22) ;
- au moins un moyen (17, 18, 19, 20) de distribution de volumes choisis d'au moins un fluide réactif, relié à l'orifice d'introduction (4a, 5a) d'au moins une chambre de préparation (4, 5) du dispositif de préparation et de distribution (1), et
- au moins un moyen (21, 22) de réception et/ou d'analyse d'un mélange d'une aliquote (a1, a2) de l'échantillon de fluide et d'un volume déterminé de réactif, relié à l'orifice de distribution (4b, 5b) de la chambre de préparation (4, 5) du dispositif de préparation et de distribution.

2. Système selon la revendication 1, **caractérisé en ce qu'**il comporte des moyens de distribution (23, 25) d'au moins un fluide de rinçage de l'organe de prélèvement, relié audit moyen (8) d'introduction d'un fluide de rinçage dans la chambre de rinçage (7) du dispositif de préparation et de distribution (1), et des moyens d'aspiration et de récupération (24, 26) d'un dit fluide de rinçage, reliés audit moyen (9) de vidange et d'évacuation d'un dit fluide de rinçage de la chambre de rinçage (7) dudit dispositif de préparation et de distribution (1).

3. Système selon l'une des revendications 1 et 2, **caractérisé en ce qu'**il comporte des moyens d'aération (14, 15) communiquant avec ledit organe de prélèvement (10).

4. Système selon l'une quelconque des revendications 1 à 3, **caractérisé en ce que** la chambre de préparation (4, 5) du dispositif (1) de préparation et de distribution est constituée par un canal traversant le dispositif formant ainsi deux orifices, respectivement, l'orifice d'introduction (4a, 5a) et l'orifice de distribution (4b, 5b) de la chambre de préparation.

5. Système selon la revendication 4, **caractérisé en ce que** la chambre de préparation (4, 5) du dispositif (1) de préparation et de distribution est constituée d'un canal orienté selon un axe de préférence perpendiculaire audit axe YY' dudit moyen de guidage (3).

6. Système selon l'une quelconque des revendications 1 à 5, **caractérisé en ce qu'**il comporte des moyens de nettoyage (7, 8, 9, 23, 24, 25, 26) d'un dit organe de prélèvement (10) introduit dans le moyen de guidage (3).

7. Système selon la revendication 6, **caractérisé en ce que** les moyens de nettoyage comportent une chambre de rinçage (7) formée dans le prolongement du moyen de guidage (3), la chambre de rinçage communicant avec au moins un moyen (8) d'introduction d'un liquide de rinçage et avec au moins un moyen (8) de vidange de ladite chambre de rinçage et d'évacuation du liquide de rinçage.

8. Système selon l'une des revendications 6 et 7, **caractérisé en ce que** les moyens de nettoyage comportent au moins un joint racleur disposé dans le prolongement du moyen de guidage (3), de manière à racler la surface externe d'un dit organe de prélèvement (10) introduit dans le moyen de guidage (3) du dispositif lors des mouvements en translation dudit organe de prélèvement dans le moyen de guidage (3) et/ou la chambre de rinçage (7).

9. Système selon l'une quelconque des revendications 1 à 8, **caractérisé en ce que** le dispositif (1) de préparation et de distribution présente une structure modulaire, chaque module supportant au moins une chambre de préparation et une portion du moyen de guidage et chaque module étant apte à être empilé avec d'autres modules dans la direction de l'axe YY'.

10. Procédé de préparation et distribution fractionnée d'un échantillon d'un fluide, notamment de sang, à l'aide d'un système (S) de distribution selon l'une quelconque des revendications 1 à 9, **caractérisé en ce que**, successivement :
a- on aspire un échantillon d'un fluide contenu dans un tube (T), dans l'organe de prélèvement (10) du système (S), l'organe de prélèvement (10) ayant été guidé préalablement en translation selon l'axe YY' au travers du dispositif (1), jusqu'à ce que l'extrémité de l'organe de prélèvement (10) s'introduise dans le tube (T) contenant le fluide,
b- on déplace ledit organe de prélèvement (10) en translation selon l'axe YY' du moyen de guidage (3) du dispositif de préparation et de distribution (1), jusqu'à amener l'orifice (12) de l'organe de prélèvement dans au moins une chambre de préparation (4, 5) du dispositif (1) de préparation et de distribution,
c- on distribue, simultanément ou non, une aliquote (a1, a2) de l'échantillon de fluide prélevé par l'orifice (12) de l'organe de prélèvement (10) et un volume déterminé d'un fluide réactif (R1, R2) dans ladite chambre de préparation (4, 5) du dispositif de préparation et de distribution (1), ladite aliquote (a1, a2) et ledit volume déterminé de fluide réactif étant mélangés puis évacués à co-courant sous la pression du fluide réactif vers un moyen (21, 22) de récupération et/ou d'analyse dudit mélange par l'orifice de distribution (4b, 5b) du dispositif de préparation et de distribution (1).

11. Procédé selon la revendication 10, **caractérisé en ce qu'**après prélèvement de l'échantillon de fluide dans un tube (T) de sang et avant distribution d'une dite aliquote (a1, a2) dans ladite chambre de préparation (4, 5) du dispositif de préparation et de distribution (1), on nettoie l'organe de prélèvement (10) ayant été en contact avec le fluide par l'intermédiaire des moyens de nettoyage (7, 8, 9, 23, 24, 25, 26) du dispositif de préparation et de distribution.

12. Procédé selon l'une des revendications 10 et 11, **caractérisé en ce que**, avant prélèvement d'un dit échantillon de fluide dans un dit tube (T), fermé par un bouchon B, on équilibre, le cas échéant, la pression à l'intérieur dudit tube, par l'intermédiaire dudit organe de prélèvement (10).

## Patentansprüche

1. System (S) zur Entnahme, Aufbereitung und fraktionierten Abgabe von Proben einer Flüssigkeit, insbesondere Blut, umfassend:
- ein Entnahmeteil (10), insbesondere eine Nadel, mit einem Ende (11), das in der Lage ist, einen Stopfen (B) eines Röhrchens (T), das eine Flüssigkeit enthält, zu durchbohren, und mindestens einer Öffnung (12), die in der Lage ist, die Belüftung des Röhrchens (T) und das Ansaugen einer in dem Röhrchen befindlichen Flüssigkeitsprobe zu ermöglichen,
- mindestens ein Mittel (13) zum Ansaugen und Ausgeben der Flüssigkeitsprobe, das mit dem Entnahmeteil (10) verbunden ist,
- mindestens eine Vorrichtung (1) zur Aufbereitung und Abgabe, umfassend:
ein Führungsmittel (3), das aus einem Kanal (3) besteht, der ausgebildet ist, um das Entnahmeteil (10) in Translation entlang einer YY'-Achse in dem Kanal (3) aufzunehmen und zu führen, bis das Ende des Entnahmeteils in das Röhrchen (T) eingeführt ist, das die Flüssigkeit enthält, und
mindestens eine Aufbereitungskammer (4, 5) zur Aufbereitung eines Aliquots einer Flüssigkeitsprobe, das in die Kammer durch das Entnahmeteil (10) in einen Strom eines geeigneten Reagenzes abgegeben wird, wobei das Führungsmittel (3) durch die Aufbereitungskammer (4, 5) hindurchgeht und mit ihr so in Verbindung steht, dass die Abgabe eines Aliquots einer Flüssigkeitsprobe in die Kammer durch das Entnahmeteil (10) in einer vorbestimmte Position desselben in dem Führungsmittel ermöglicht wird, wobei die Aufbereitungskammer (4, 5) eine Einleitungsöffnung (4a, 5a) von mindestens einem Reagenz (R1, R2) in die Kammer zum Mischen des Reagenzes mit einem Aliquot und mindestens eine Abgabeöffnung (4b, 5b) zur Abgabe der aus dem Aliquot und dem Reagenz gebildeten Mischung an Sammel- und/oder Analysemittel (21, 22) umfasst,
- mindestens ein Mittel (17, 18, 19, 20) zum Abgeben ausgewählter Volumina mindestens einer reaktiven Flüssigkeit, das mit der Einführungsöffnung (4a, 5a) mindestens einer Aufbereitungskammer (4, 5) der Vorrichtung zur Aufbereitung und Abgabe (1) verbunden ist, und
- mindestens ein Mittel (21, 22) zum Aufnehmen und/oder Analysieren eines Gemisches aus einem Aliquot (a1, a2) der Flüssigkeitsprobe und einem bestimmten Volumen an Reagenz, das mit der Abgabeöffnung (4b, 5b) der Aufbereitungskammer (4, 5) der Vorrichtung zur Aufbereitung und Abgabe verbunden ist.

2. System gemäß Anspruch 1, **dadurch gekennzeichnet, dass** es Mittel zum Abgeben (23, 25) mindestens einer Spülflüssigkeit von dem Entnahmeteil, die mit dem Mittel (8) zum Einführen einer Spülflüssigkeit in die Spülkammer (7) der Vorrichtung zur Aufbereitung und Abgabe (1) verbunden sind, und Mittel zum Ansaugen und Sammeln (24, 26) einer solchen Spülflüssigkeit aufweist, die mit dem Mittel (9) zum Entleeren und Abführen einer solchen Spülflüssigkeit der Spülkammer (7) der Vorrichtung zur Aufbereitung und Abgabe (1) verbunden sind.

3. System gemäß einem der Ansprüche 1 und 2, **dadurch gekennzeichnet, dass** es Belüftungsmittel (14, 15) umfasst, die mit dem Entnahmeteil (10) in Verbindung stehen.

4. System gemäß einem der Ansprüche 1 bis 3, **dadurch gekennzeichnet, dass** die Aufbereitungskammer (4, 5) der Vorrichtung (1) zur Aufbereitung und Abgabe durch einen Kanal gebildet wird, der die Vorrichtung durchquert, wodurch zwei Öffnungen gebildet werden, jeweils die Einführungsöffnung (4a, 5a) und die Abgabeöffnung (4b, 5b) der Aufbereitungskammer.

5. System gemäß Anspruch 4, **dadurch gekennzeichnet, dass** die Aufbereitungskammer (4, 5) der Vorrichtung (1) zur Aufbereitung und Abgabe aus einem Kanal besteht, der entlang einer Achse orientiert ist, die vorzugsweise senkrecht zur YY'-Achse des Führungsmittels (3) verläuft.

6. System gemäß einem der Ansprüche 1 bis 5, **dadurch gekennzeichnet, dass** es Mittel zum Reinigen (7, 8, 9, 23, 24, 25, 26) eines solchen in das Führungsmittel (3) eingeführten Entnahmeteils (10) aufweist.

7. System gemäß Anspruch 6, **dadurch gekennzeichnet, dass** die Mittel zum Reinigen eine Spülkammer (7) umfassen, die in Verlängerung des Führungsmittels (3) ausgebildet ist, wobei die Spülkammer mit mindestens einem Mittel (8) zum Einführen einer Spülflüssigkeit und mit mindestens einem Mittel (8) zum Entleeren der Spülkammer und zum Abführen der Spülflüssigkeit in Verbindung steht.

8. System gemäß einem der Ansprüche 6 und 7, **dadurch gekennzeichnet, dass** die Reinigungsmittel mindestens eine Abstreiferdichtung umfassen, die in Verlängerung des Führungsmittels (3) angeordnet ist, um die Außenfläche eines solchen Entnahmeteils (10), das in das Führungsmittel (3) der Vorrichtung eingeführt ist, während der Translationsbewegungen des Entnahmeteils in dem Führungsmittel (3) und/oder der Spülkammer (7) abzustreifen.

9. System gemäß einem der Ansprüche 1 bis 8, **dadurch gekennzeichnet, dass** die Vorrichtung (1) zur Aufbereitung und Abgabe eine modulare Struktur aufweist, wobei jedes Modul mindestens eine Aufbereitungskammer und einen Abschnitt des Führungsmittels trägt und jedes Modul geeignet ist, mit anderen Modulen in Richtung der YY'-Achse gestapelt zu werden.

10. Verfahren zur Aufbereitung und fraktionierten Abgabe einer Flüssigkeitsprobe, insbesondere Blut, unter Verwendung eines Abgabesystems (S) gemäß einem der Ansprüche 1 bis 9, **dadurch gekennzeichnet, dass** nacheinander:
a- eine Flüssigkeitsprobe, die in einem Röhrchen (T) enthalten ist, in das Entnahmeteil (10) des Systems (S) eingezogen wird, wobei das Entnahmeteil (10) zuvor entlang der YY'-Achse durch die Vorrichtung (1) geführt wird, bis das Ende des Entnahmeteils (10) in das die Flüssigkeit enthaltende Röhrchen (T) eingeführt ist,
b- das Entnahmeteil (10) entlang der YY'-Achse des Führungsmittels (3) der Vorrichtung zur Aufbereitung und Abgabe (1) verschoben wird, bis die Öffnung (12) des Entnahmeteils in mindestens eine Aufbereitungskammer ('4, 5) der Vorrichtung (1) zur Aufbereitung und Abgabe gebracht ist,
c- ein Aliquot (a1, a2) der Flüssigkeitsprobe, die durch die Öffnung (12) des Entnahmeteils (10) entnommen wurde, und ein bestimmtes Volumen einer reaktiven Flüssigkeit (R1, R2) gleichzeitig oder nicht gleichzeitig in die Aufbereitungskammer (4, 5) der Vorrichtung zur Aufbereitung und Abgabe (1) abgegeben werden, wobei das Aliquot (a1, a2) und das bestimmte Volumen der reaktiven Flüssigkeit gemischt und anschließend unter dem Druck der reaktiven Flüssigkeit im Gleichstrom zu einem Mittel (21, 22) zum Sammeln und/oder Analysieren des Gemisches durch die Abgabeöffnung (4b, 5b) der Vorrichtung zur Aufbereitung und Abgabe (1) abgeführt werden.

11. Verfahren gemäß Anspruch 10, **dadurch gekennzeichnet, dass** nach der Entnahme der in einem Blutröhrchen (T) befindlichen Flüssigkeitsprobe und vor der Abgabe eines Aliquots (a1, a2) in der Aufbereitungskammer (4, 5) der Vorrichtung zur Aufbereitung und Abgabe (1) das mit der Flüssigkeit in Berührung gekommene Entnahmeteil (10) mit Hilfe der Reinigungsmittel (7, 8, 9, 23, 24, 25, 26) gereinigt wird.

12. Verfahren gemäß einem der Ansprüche 10 und 11, **dadurch gekennzeichnet, dass** vor der Entnahme einer solchen Flüssigkeitsprobe aus einem solchen Röhrchen (T), das durch einen Stopfen B verschlossen ist, gegebenenfalls der Druck in dem Röhrchen über das Entnahmeteil (10) ausgeglichen wird.

## Claims

1. A system (S) for sampling, preparing, and fractioned dispensing of samples of a fluid, in particular blood, comprising:
• a sample-taker member (10), in particular a needle, having an end (11) suitable for piercing a stopper (B) of a tube (T) containing a fluid, and at least one orifice (12) suitable for enabling air to be delivered to said tube (T) and for sucking up a sample of fluid in the tube;
• at least one means (13) for sucking up and delivering the sample of fluid, said means being connected to the sample-taker member (10);
• at least one preparation and dispensing device (1) comprising:
guide means (3) constituted by a channel (3) which is adapted to receive the sample-taker member (10) and for guiding it in translation within the device along an axis YY' through the channel (3) until the end of the sample-taker member is introduced into the tube (T) containing the fluid;
at least one preparation chamber (4, 5) enabling an aliquot of a sample of fluid dispensed in the chamber by said sample-taker member (10) to be prepared in a stream of an appropriate reagent, the guide means (3) passing through the preparation chamber (4, 5) and communicating therewith in such a manner as to enable a said aliquot of a said fluid sample to be dispensed into the chamber by the sample-taker member (10) in a determined position thereof within the guide means, the preparation chamber (4, 5) including an introduction orifice (4a, 5a) for introducing at least one reagent (R1, R2) into the chamber for mixing the reagent with a said aliquot, and at least one dispensing orifice (4b, 5b) for dispensing the mixture formed by said aliquot and said reagent to recovery and/or analysis means (21, 22);
• at least one means (17, 18, 19, 20) for dispensing selected volumes of at least one reagent fluid, said means being connected to the introduction orifice (4a, 5a) of at least one preparation chamber (4, 5) of the preparation and dispensing device (1); and
• at least one means (21, 22) for receiving and/or analyzing a mixture of an aliquot (a1, a2) of the sample of fluid and a determined volume of reagent, said means being connected to the dispensing orifice (4b, 5b) of the preparation chamber (4, 5) of the preparation and dispensing device.

2. A system according to claim 1, **characterized in that** it comprises means (23, 25) for dispensing at least one rinsing fluid for rinsing the sample-taker member, said means being connected to said means (8) for introducing a rinsing fluid into the rinsing chamber (7) of the preparation and dispensing device (1), and means (24, 26) for sucking up and recovering a said rinsing fluid, said means being connected to said means (9) for discharging and removing a said rinsing fluid from the rinsing chamber (7) of said preparation and dispensing device (1).

3. A system according to claim 1 or 2, **characterized in that** it comprises air flow means (14, 15) communicating with said sample-taker member (10).

4. A system according to any one of claims 1 to 3, **characterized in that** the preparation chamber (4, 5) of the preparation and dispensing device (1) is constituted by a channel passing through the device, thereby forming two orifices, respectively the introduction orifice (4a, 5a) and the dispensing orifice (4b, 5b) of the preparation chamber.

5. A system according to claim 4, **characterized in that** the preparation chamber (4, 5) of the preparation and dispensing device (1) is constituted by a channel oriented along an axis that is preferably perpendicular to said axis YY' of said guide means (3).

6. A system according to any one of claims 1 to 5, **characterized in that** it comprises cleaner means (7, 8, 9, 23, 24, 25, 26) for cleaning a said sample-taker member (10) introduced into the guide means (3).

7. A system according to claim 6, **characterized in that** the cleaner means comprise a rinsing chamber (7) formed in line with the guide means (3), the rinsing chamber communicating with at least one means (8) for introducing a rinsing liquid and with at least one means (8) for discharging said rinsing chamber and removing the rinsing liquid.

8. A system according to claim 6 or 7, **characterized in that** the cleaner means include at least one wiper gasket disposed in line with the guide means (3) so as to wipe the outside surface of a said sample-taker member (10) introduced into the guide means (3) of the device during the movements in translation of said sample-taker member in the guide means (3) and/or the rinsing chamber (7).

9. A system according to any one of claims 1 to 8, **characterized in that** the preparation and dispensing device (1) presents a modular structure, each module supporting at least one preparation chamber and a portion of the guide means, and each module being suitable for being stacked with other modules in the direction of the axis YY'.

10. A method for the preparation and fractioned dispensing of a sample of a fluid, in particular blood, using a distribution system (S) according to any one of claims 1 to 9, the method being **characterized by** the following successive steps:
a) sucking up a sample of a fluid contained in a tube (T) into the sample-taker member (10) of the system (S), the sample-taker member (10) previously being guided to move in translation along the axis YY' through the device (1) until the end of the sample-taker member (10) is introduced into the tube (T) containing the fluid;
b) moving said sample-taker member (10) in translation along the axis YY' of the guide means (3) of the preparation and dispensing device (1) until the orifice (12) of the sample-taker member (10) is brought into at least one preparation chamber (4, 5) of the preparation and dispensing device (1); and
c) simultaneously or otherwise dispensing an aliquot (a1, a2) of the taken fluid sample via the orifice (12) of the sample-taker member (10) and a determined volume of a reagent fluid (R1, R2)) into the preparation chamber (4, 5) of the preparation and dispensing device (1), said aliquot (a1, a2) and said determined volume of reagent fluid being mixed and then discharged together under the pressure of the reagent fluid to means (21, 22) for recovering and/or analyzing said mixture via the dispensing orifice (4b, 5b) of the preparation and dispensing device (1).

11. A method according to claim 10, **characterized in that** after the fluid sample has been taken from the tube (T) of blood and before a said aliquot (a1, a2) has been dispensed into said preparation chamber (4, 5) of the preparation and dispensing device (1), the sample-taker member (10) that has been in contact with the fluid is cleaned by using the cleaner means (7, 8, 9, 23, 24, 25, 26) of the preparation and dispensing device.

12. A method according to claim 10 or 11, **characterized in that**, prior to taking a said fluid sample from a said tube (T) closed by a stopper B, and where appropriate, the pressure inside said tube is balanced by using said sample-taker member (10).
